## Europäisches Patentamt
## European Patent Office
## Office européen des brevets

(11) Numéro de publication: **0 046 110**
**B1**

(12) **FASCICULE DE BREVET EUROPEEN**

(45) Date de publication du fascicule du brevet:
**10.04.85**

(51) Int. Cl.⁴: **G 01 R 29/033,** G 07 C 1/00, A 61 B 5/02

(21) Numéro de dépôt: **81401252.2**

(22) Date de dépôt: **03.08.81**

(54) **Dispositif d'obtention de l'histogramme des distances dans le temps entre des événements successifs.**

(30) Priorité: **07.08.80 FR 8017708**

(43) Date de publication de la demande:
**17.02.82 Bulletin 82/7**

(45) Mention de la délivrance du brevet:
**10.04.85 Bulletin 85/15**

(84) Etats contractants désignés:
**DE GB IT NL**

(73) Titulaire: **Urien, Michel, rue du Park Moan Brélevenez, F-22300 Lannion (FR)**
Titulaire: **Theron, Guy Marie Pierre Jean, 42 rue de Lorraine, F-22300 Lannion (FR)**

(72) Inventeur: **Urien, Michel, rue du Park Moan Brélevenez, F-22300 Lannion (FR)**
Inventeur: **Theron, Guy Marie Pierre Jean, 42 rue de Lorraine, F-22300 Lannion (FR)**

(74) Mandataire: **Le Guen, Louis François, 13, rue Emile Bara BP 91, F-35802 Dinard Cedex (FR)**

(56) Documents cités:
**DE - A - 2 750 646**

**INSTRUMENTS AND EXPERIMENTAL TECHNIQUES, no. 6, novembre/décembre 1970, Consultants Bureau, New York, US, E.I. KRASOVSKII et al.: "Digital analyzer of the time interval between pulses of a random process", pages 1615-1616**
**INSTRUMENTS & CONTROL SYSTEMS, vol. 40, no. 19, novembre 1967, V. CAGGIANO et al.: "Delay line memory in physiological data analysis", pages 93-97**
**ELEKTRONIK, vol. 24, no. 2, 1975, E. ENGER "Intervallmeter mit automatischer Bereichseinstellung", pages 61-65**

ACTORUM AG

## Description

La présente invention concerne un dispositif permettant d'obtenir l'histogramme des distances dans le temps entre des événements successifs.

Dans les dispositifs ou les appareillages de mesure connus de ce genre, on compte simplement le nombre d'événements apparaissant durant une période de temps donnée, puis on calcule le rapport entre le nombre d'événements apparus et le nombre d'unités de temps de la période donnée.

De tels dispositifs connus ne permettent pas d'effectuer une analyse fine des événements apparaissant par paquets ou apparaissant regroupés dans le temps.

D'autres systèmes d'analyse connus ne permettent que l'enregistrement des distances entre événements successifs, si bien que l'histogramme des distances ne peut être obtenu qu'en temps différé.

Des exemples de dispositifs connus sont décrits dans l'article de E.I. KRASOVSKII et al. paru dans «INSTRUMENTS AND EXPERIMENTAL TECHNIQUES», N° 6, NOV.–DEC. 1970 et intitulé: «DIGITAL ANALYZER OF THE TIME INTERVAL BETWEEN PULSES OF A RANDOM PROCESS» et dans celui de V. CAGGIANO et al. paru dans «INSTRUMENTS & CONTROL SYSTEMS» Vol. 40, N° 19, NOV. 1967 et intitulé: «DELAY LINE MEMORY IN PHYSIOLOGICAL DATA ANALYSIS».

Un objectif de la présente invention consiste à prévoir un dispositif capable d'établir en temps réel, d'une manière continue, sans perte d'informations, les histogrammes des distances entre événements successifs.

Un autre objectif de l'invention consiste à prévoir un dispositif capable d'effectuer l'analyse fine et le classement d'événements apparaissant regroupés par paquets.

Un autre objectif de l'invention consiste à prévoir un tel dispositif qui puisse être associé à un circuit approprié permettant de connaître l'apparition des paquets d'événements, et à un enregistreur de résultats.

Le dispositif conforme à l'invention est défini et décrit par la revendication 1.

Les caractéristiques de l'invention mentionnées ci-dessus, ainsi que d'autres, apparaîtront plus clairement à la lecture de la description d'un exemple de réalisation, ladite description étant faite en relation avec les dessins joints, parmi lesquels:

la fig. 1 est un bloc-diagramme général d'un dispositif suivant l'invention,

la fig. 2 est une vue schématique de l'ensemble des circuits de comptage et de mémoire du dispositif de la fig. 1,

les figs 3a à 3g sont des diagrammes temporels illustrant le fonctionnement du dispositif de la fig. 1, et

les figs 4 à 7 sont des schémas-blocs plus détaillés de circuits du dispositif de la fig. 1.

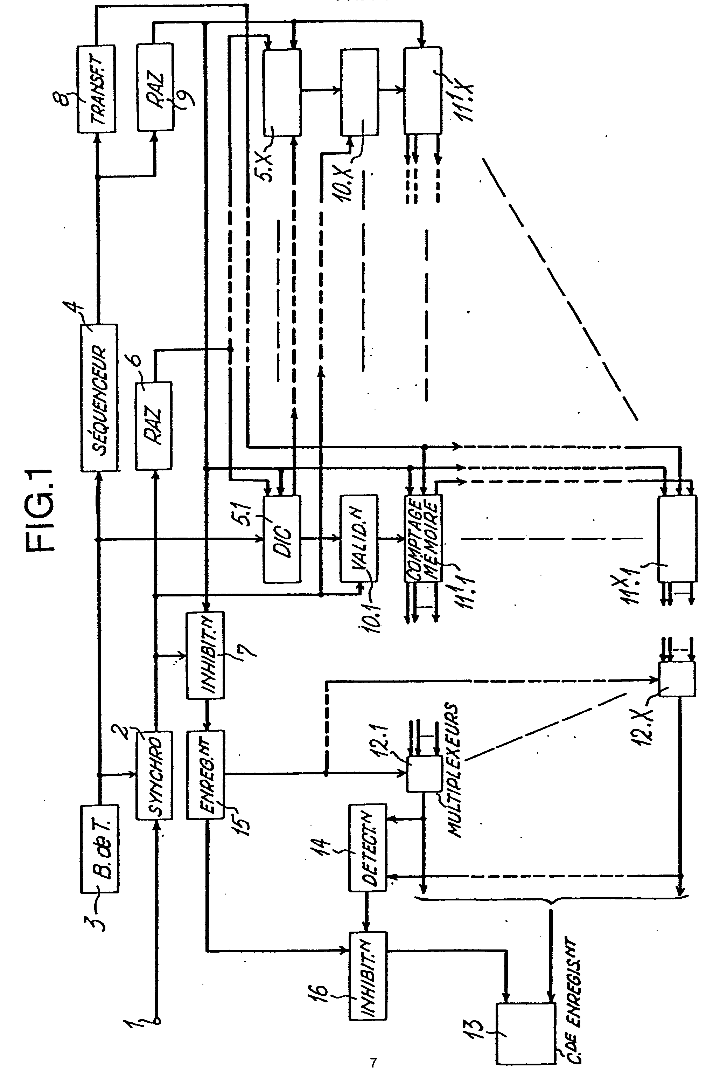

A la fig. 1, les événements dont les distances sont à analyser sont appliqués, sous forme de signaux logiques, à la borne 1 qui est reliée à une entrée d'un circuit de synchronisation 2 dont la seconde entrée est reliée à la sortie d'une base de temps 3. La sortie de la base de temps 3 est encore reliée à l'entrée d'un séquenceur 4 et à l'entrée d'un circuit de détermination d'intervalle de comptage ou circuit DIC 5.1. La sortie du circuit de synchronisation 2 est reliée à l'entrée d'un premier circuit de remise à zéro ou circuit RAZ 6 et à l'entrée d'un circuit d'inhibition 7. La sortie du séquenceur 4 est reliée à l'entrée d'un circuit de transfert 8 et à l'entrée d'un second circuit de remise à zéro ou circuit RAZ 9.

En fait, le dispositif comprend une batterie de circuits DIC 5.1 à 5.X, l'entrée de comptage d'un circuit DIC 5.j étant reliée à la sortie de débordement du circuit DIC 5.(j–1). La sortie du circuit RAZ 6 est reliée, en parallèle, aux entrées de remise à zéro des circuits DIC 5.1 à 5.X.

Le dispositif comprend encore une batterie de circuit de validation 10.1 à 10.X. Chaque entrée de signal d'un circuit de validation 10.1 à 10.X est respectivement reliée à la sortie de signal du circuit DIC 5.1 à 5.X correspondant.

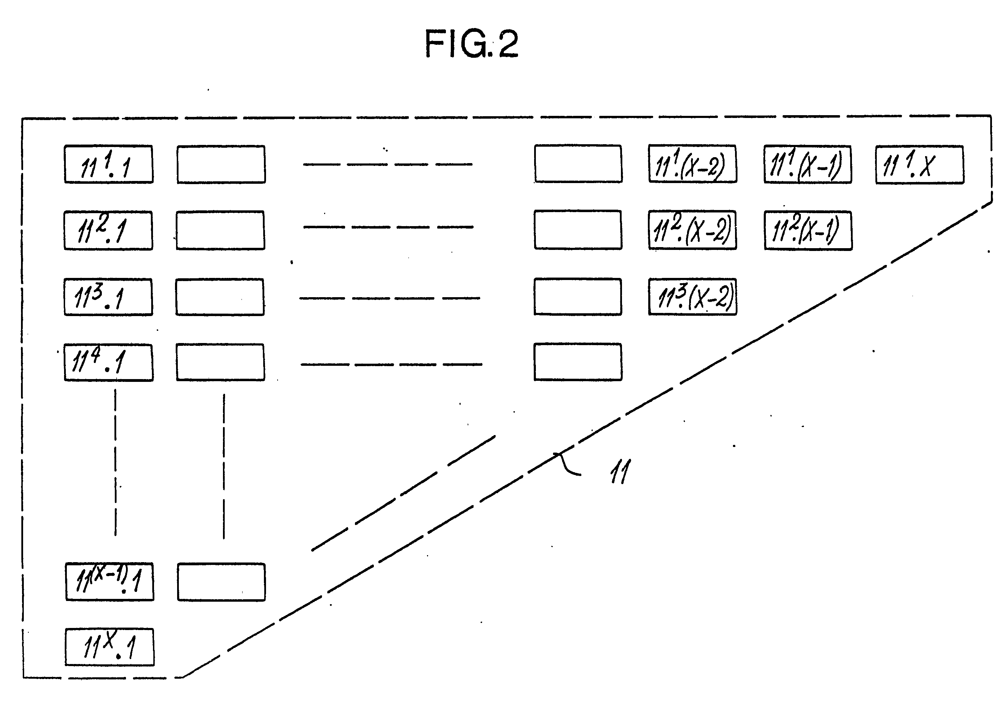

Le dispositif comprend encore un ensemble 11 de circuit de comptage et de mémoire qui est montré plus en détail à la fig. 2. Cet ensemble 11 comprend les circuits $11^1.1$ à $11^1.X$, $11^2.1$ à $11^2.(X–1)$, $11^3.1$ à $11^3.(X–2)$, ..., $11^{(X–1)}.1$ et $11^{(X–1)}.2$, et $11^X.1$. Les entrées des circuits $11^1.1$ à $11^1.X$ sont respectivement reliées aux sorties des circuits 10.1 à 10.X. Les entrées des circuits $11^2.1$ à $11^2.(X–1)$ sont respectivement reliées aux sorties des circuits $11^1.1$ à $11^1.(X–1)$, et ainsi de suite l'entrée du circuit $11^X.1$ étant reliée à la sortie du circuit $11^{(X–1)}.1$.

Par ailleurs, les sorties des circuits $11^1.1$ à $11^1.X$ sont reliées en parallèle aux entrées de signal d'un multiplexeur 12.1. De même, les sorties des circuits $11^2.1$ à $11^2.(X–1)$ sont reliées, en parallèle, à l'entrée d'un multiplexeur 12.2, et ainsi de suite, la sortie du circuit $11^X.1$ étant reliée à l'entrée du multiplexeur 12.X.

Les sorties des multiplexeurs 12.1 à 12.X sont respectivement reliées aux entrées correspondantes d'un appareil d'enregistrement 13. Elles sont également reliées, en parallèle, à l'entrée d'un circuit de détection 14.

La sortie du circuit de transfert 8 est reliée en parallèle aux entrées de commande de transfert des circuits $11^1.1$ à $11^X.1$. La sortie du circuit RAZ 9 est également reliée, en parallèle, aux entrées de remise à zéro des circuits $11^1.1$ à $11^X.1$.

Enfin, la sortie du circuit de synchronisation 2 est encore reliée aux entrées de validation des circuits de validation 10.1 à 10.X. L'entrée de validation du circuit d'inhibition 7 est reliée à la sortie du circuit RAZ 9 et sa sortie est reliée à l'entrée de validation d'un circuit de commande d'enregistrement 15 dont une sortie est reliée à l'entrée d'un circuit d'inhibition 16 et dont l'autre sortie est reliée, en parallèle, aux entrées de commande des multiplexeurs 12.1 à 12.X. La sortie du circuit de détection 14 est reliée à l'entrée d'inhibition du circuit d'inhibition 16 dont la sortie est reliée à l'entrée de commande de l'appareil d'enregistrement 13.

La base de temps 3 est constituée, par exemple, par un oscillateur à quartz délivrant des impulsions à une fréquence qui est au moins égale à la fréquence maximale à laquelle les événements à dénombrer peuvent se présenter.

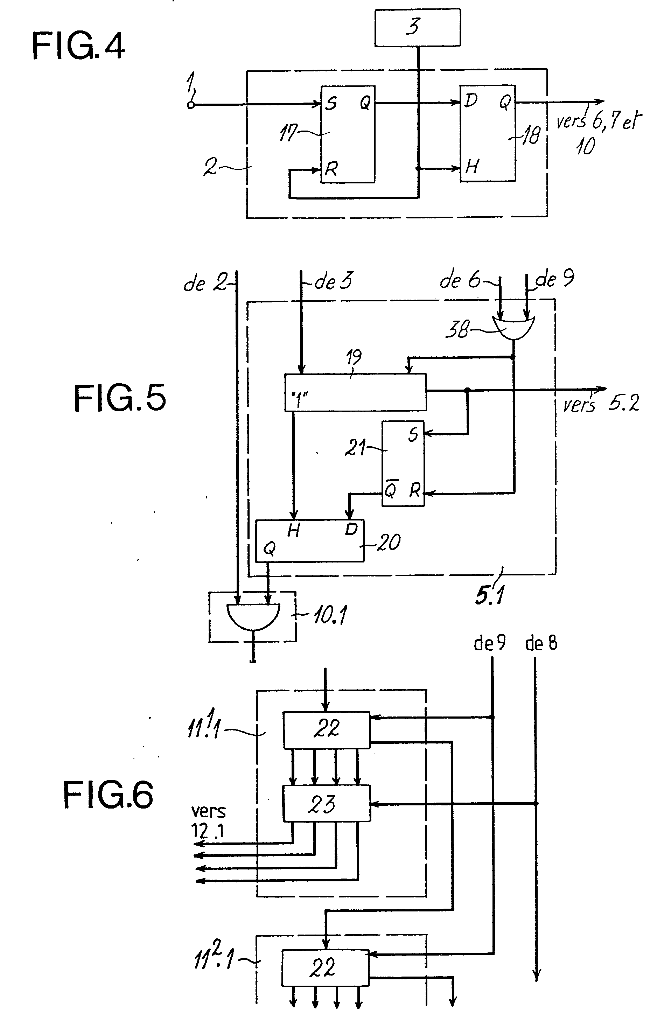

Le circuit de synchronisation 2 est un circuit classique qui, pour chaque signal appliqué en 1, délivre à sa sortie un signal en phase avec l'impulsion de base de temps qui suit immédiatement l'application du signal en 1. En pratique, le circuit 2 se compose d'une bascule RS 17, fig. 4, dont l'entrée S est reliée à l'entrée 1, dont la sortie Q est reliée à l'entrée D d'une bascule D 18 dont l'entrée H est reliée à la sortie de la base de temps 3. L'entrée R de 17 est également reliée à la sortie de 3. Ainsi la bascule 17 est mise au travail par chaque signal reçu de 1 et la bascule 18 recopie l'état de 17 et est lue à chaque impulsion reçue de la base de temps, la première bascule étant remise à l'état de repos à chaque lecture de la seconde bascule.

Le séquenceur 4 est un compteur qui fonctionne comme un diviseur de fréquence en délivrant périodiquement une impulsion aux circuits 8 et 9 afin de déclencher l'enregistrement dans l'appareil 13 des données en mémoire dans les circuits $11^1.1$ à $11^X.1$. La capacité du compteur du séquenceur 4, qui détermine la fréquence des impulsions délivrées à 8 et 9, est choisie en fonction de la vitesse maximale d'enregistrement de l'appareil enregistreur 13.

Le circuit de transfert 8 est un circuit monostable, fonctionnant en circuit à retard et qui délivre une impulsion, dite de transfert, un certain temps t après le signal émis par 4.

Le circuit RAZ 9 est également un circuit monostable, remplissant également le rôle d'un circuit à retard. Le retard apporté par 9 est supérieur à celui apporté par 8, la différence de temps entre les deux correspondants aux temps alloués pour effectuer le transfert des données des circuits $11^1.1$ à $11^X.1$ vers les multiplexeurs 12.1 à 12.X.

Le circuit RAZ 6 est également un circuit monostable dont le rôle est d'apporter un retard qui couvre le temps d'enregistrement d'un événement dans un circuit $11^1.1$ à $11^1.X$.

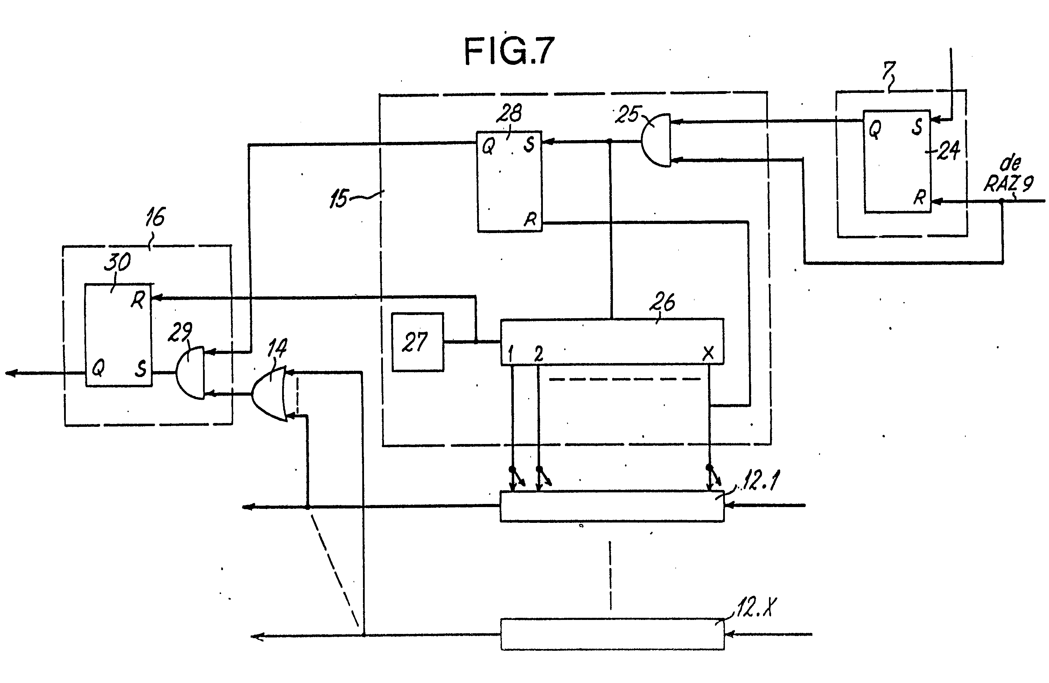

Les circuits DIC 5.1 à 5.X sont des compteurs diviseurs par 10, montés en cascade. Ces compteurs déterminent donc chacun des fenêtres temporelles de durées multiples. Ainsi, le circuit DIC 5.1 détermine une fenêtre de durée égale à dix intervalles unitaires, un intervalle unitaire correspondant à la période de la base de temps 3. Le circuit DIC 5.2 détermine une fenêtre de durée allant de «11» à «100» intervalles unitaires, suivant l'instant initial, et ainsi de suite le circuit DIC 5.X déterminant une fenêtre allant de $10^{X-1}$ à $10^X$ intervalles unitaires. Les circuits DIC 5.1 à 5.X comportent chacun une sortie d'état qui est à l'état de travail, quand une impulsion de comptage est appliquée au compteur au cours du premier cycle de comptage suivant une remise à zéro par le circuit 6 ou le circuit 9. En pratique, à titre d'exemple, le circuit DIC 5.1, fig. 5, se compose d'un compteur par dix 19, d'une bascule D 20 dont l'entrée H est reliée à la sortie de compte «1» de 19 et dont l'entrée D est reliée à la sortie Q̄ d'une bascule RS 21 dont l'entrée S est reliée à la sortie de débordement de 19 et l'entrée R à la sortie d'une porte OU 38 dont une entrée est reliée à la sortie de 6 et l'autre à la sortie de 9. L'entrée de signal de 19 est reliée à la base de temps 3. L'entrée RAZ de 19 est reliée à la sortie de 38.

Les circuits de validation 10.1 à 10.X sont constitués chacun par des circuits ET dont une entrée est reliée à la sortie du circuit 2 et l'autre entrée à la sortie du compteur 5.1 à 5.X correspondant.

Comme le montre la fig. 6, le circuit $11^1.1$ comprend un compteur 22 dont l'entrée de signal est reliée à la sortie du circuit de validation 10.1. L'entrée de remise à zéro du compteur 22 est reliée à la sortie du circuit RAZ 9, et sa sortie de débordement est reliée à l'entrée de signal du compteur correspondant 22 du circuit $11^2.1$. Les sorties de comptage du compteur 22 sont reliées aux entrées correspondantes d'une mémoire 23 dont l'entrée de commande de transfert est reliée à la sortie du circuit de transfert 8. Les sorties de la mémoire 23 sont reliées aux entrées correspondantes du multiplexeur correspondant. Bien entendu, les autres circuits $11^1.2$ à $11^X.1$ comprennent également chacun un compteur 22 et une mémoire 23. Pour tous les circuits $11^1.1$ à $11^1.X$, les entrées de signal des compteurs 22 sont respectivement reliées aux sorties des circuits de validation correspondant. Pour les autres circuits $11^2.1$ à $11^X.1$, les entrées de signal des compteurs 22 sont respectivement reliées aux sorties de débordement des compteurs 22 des circuits de rangs immédiatement inférieurs.

Comme le montre la fig. 2, l'ensemble 11 est constitué par une matrice triangulaire de circuits, dont les circuits de rang 1 sont au nombre de X, les circuits de rang 2 au nombre de (X–1);.., et les circuits de rang X au nombre de 1.

Chaque compteur 22 compte jusqu'à 10, si bien que les compteurs 22 d'une colonne de la matrice de la fig. 2 forment une cascade de diviseurs par dix. Chaque mémoire 23 est constituée par quatre bascules D. Les sorties de signal des compteurs 22 sont évidemment au nombre de quatre pour le codage binaire d'un nombre de 0 à 9. Le transfert du compte contenu dans un compteur 22 vers sa mémoire associée 23 est effectué à chaque impulsion émise par le circuit 8. Les comptes ainsi transférés restent emmagasinés dans les mémoires 23 jusqu'à l'ordre de transfert suivant.

Le circuit d'inhibition 7 est une bascule qui est mise à l'état de repos par la sortie du circuit RAZ. Dès qu'un signal d'événement est délivré par le circuit 2, la bascule 7 est mise à l'état de travail.

Le circuit d'inhibition 7 peut être une bascule RS 24, fig. 7, dont l'entrée R est reliée à la sortie du circuit RAZ 9 et dont l'entrée S est reliée à la sortie du circuit 2. Dès qu'un signal d'événement est délivré par le circuit 2, la sortie Q de la bascule 23 est activée.

Le circuit de commande d'enregistrement 15, fig. 7, comprend une porte ET 25 dont une entrée est reliée à la sortie Q de la bascule 24 et l'autre entrée à la sortie du circuit RAZ 9. La sortie de la porte ET 25 est reliée à l'entrée de remise à zéro d'un circuit de séquencement 26 qui est constitué par un compteur dont les sorties commandent les connexions des multiplexeurs 12.1 à 12.X. L'entrée de comptage du compteur 26 est reliée à la sortie d'une base de temps 27 dont la fréquence est choisie en fonction des caractéristiques de l'appareil enregistreur. La sortie de la porte ET 25 est encore reliée à l'entrée S d'une bascule RS 28 dont l'entrée R est reliée à la dernière sortie X du compteur 26.

Le circuit de détection 14 est une porte OU dont les entrées sont respectivement reliées aux sorties des multiplexeurs 12.1 à 12.X.

Le circuit d'inhibition 16 comprend une porte ET 29 dont une entrée est reliée à la sortie Q de la bascule 28 et l'autre entrée à la sortie de la porte OU 14. Il comprend encore une bascule RS 30 dont l'entrée S est reliée à la sortie de la porte ET 29 et l'entrée R à la sortie de la base de temps 27. La sortie Q de la bascule 30 est reliée à l'entrée de commande d'enregistrement de l'appareil 13.

On va tout d'abord décrire le fonctionnement des circuits concernant l'enregistrement proprement dit. On suppose qu'un ordre de transfert a été délivré par le circuit 8, ce qui entraîne, dans chaque mémoire 23 d'un circuit de validation, le transfert du compteur 22 dans cette mémoire. Un peu plus tard, le circuit RAZ 9 émet un signal qui remet les compteurs 22 au repos et qui, par ailleurs, déclenche le fonctionnement du circuit de séquencement 26 du circuit 15 par l'intermédiaire de la porte 25, la bascule 24 étant supposée avoir été mise au travail précédemment. Le circuit 26 fait fonctionner l'ensemble des multiplexeurs 12.1 à 12.X, qui commence par relier le circuit $11^1.X$ à l'appareil 13. Si le contenu de $11^1.X$ n'est pas nul, la porte OU 14 fait passer un signal non nul qui met au travail la bascule 30, par l'intermédiaire de la porte ET 29 dont l'autre entrée est activée par 25 à travers 28. La sortie Q de la bascule 30 commande l'enregistrement effectif du contenu de la mémoire 23 de $11^1.X$ dans 13 par l'intermédiaire de 12.1. A l'impulsion suivante de la base de temps 27, la bascule 30 est remise au repos et les multiplexeurs 12.1 et 12.2 connectent les circuits $11^1.(X{-}1)$ et $11^2.(X{-}1)$. Si le contenu des mémoires de ces circuits ne sont pas simultanément nuls, la porte 14 met 30 au travail et l'enregistrement se poursuit comme précédemment. La suite des phases d'enregistrement se poursuit ainsi jusqu'à ce que les multiplexeurs 12.1 à 12.X connectent les circuits $11^1.1$ à $11^X.1$. Si, dans l'une des phases, toutes les mémoires 23 d'une colonne sont vides, la porte OU 14 ne fait pas basculer la bascule 30 si bien que l'enregistrement est inhibé et que l'imprimante du circuit 13 n'avance pas. Quand le compteur 26 a atteint le compte X, la bascule 28 est remise au repos ce qui, un instant plus tard, inhibe l'enregistrement. Le temps de bascule de 28 est assez long pour couvrir l'opération d'enregistrement des contenus des mémoires dans 13.

Par ailleurs, si dans un intervalle de temps défini par le séquenceur 4, aucun signal d'événement n'a été délivré par 2, la bascule 24 de 7 reste au repos, ce qui empêche le déclenchement du circuit 15.

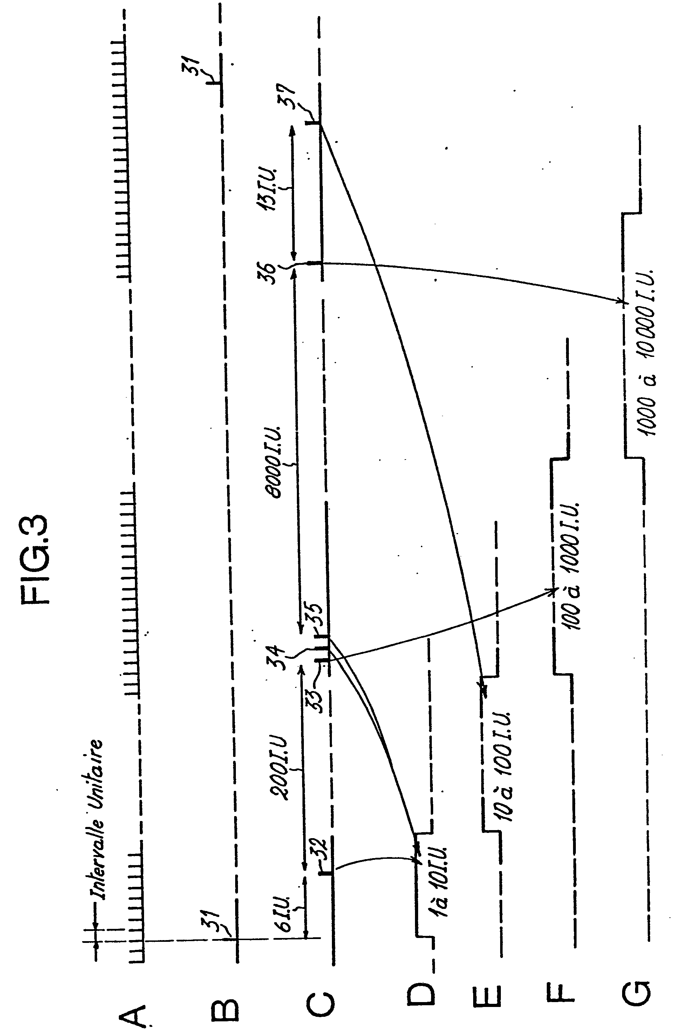

On va maintenant, en se référant aux diagrammes temporels des figs 3a à 3g, montrer comment les événements sont comptabilisés dans les compteurs 22 des circuits $11^1.1$ à $11^1.X$. A la fig. 3a, on a représenté les impulsions délivrées par la base de temps 3. A la fig. 3b, on a représenté les impulsions délivrées par le séquenceur 4, ces impulsions définissant, entre autres, les origines de comptage. A la fig. 3c, on a représenté une suite de signaux délivrés par le circuit 2, c'est-à-dire de signaux qui reflètent des événements appliqués, sous forme de signaux électriques, au dispositif de l'invention. La fig. 3c montre un événement isolé 32 délivré 6 intervalles unitaires après la première impulsion 31, fig. 3b, les intervalles unitaires étant définis par l'intervalle entre les impulsions de la fig. 3a, puis un groupe d'événements 33, 34 et 35, délivré 200 intervalles unitaires et séparés les uns des autres par un intervalle unitaire, puis un intervalle 36 délivré 8000 intervalles unitaires après 35, et enfin un dernier événement 37 avant l'impulsion 31 suivante et délivré 13 intervalles unitaires après 36.

Quand 32 survient, le compteur 19 de DIC 5.1 n'a pas encore débordé, donc la bascule 20 est au travail ce qui entraîne le passage de 32 à travers la porte ET de 10.1 vers le compteur 22 de $11^1.1$.

Quand 33 survient, le compteur 19 de DIC 5.3 n'a pas encore débordé, alors que ceux de DIC 5.1 et 5.2 l'ont fait. Donc on a un comptage dans le compteur 22 de $11^1.3$.

Quand 34 survient, à l'intervalle unitaire suivant 33, les compteurs 19 ont été remis à zéro si bien que la bascule 20 de DIC 5.1 est à nouveau mise au travail par l'arrivée de 34 et le compteur 22 de $11^1.1$ est incrémenté. Il en est de même à l'arrivée de 35.

Quand 36 survient, le compte du compteur 22 de $11^1.4$ augmente d'une unité.

Quand 37 survient, le compte du compteur 22 de $11^1{\cdot}2$ augmente d'une unité.

On a donc dans les compteurs 22 de $11^1.1$ à $11^1.4$, les comptes respectifs suivants: «3», «1», «1», «1». Comme on l'a dit ci-dessus, les circuits de comptage et de mémoire sont arrangés en colonnes et les compteurs 22 ont une capacité de 10, ce qui permet, par l'intermédiaire des multiplexeurs 12.1 à 12.X d'obtenir directement une écriture en décimal par colonne.

Un enregistrement par l'appareil 13 se présente donc sous la forme suivante:

```
          0
         03
        154
       0010
      25312
     003427
    1032470
   00000420
```

## Revendications

1) Dispositif permettant d'obtenir l'histogramme des distances dans le temps entre des événements successifs fournis par une source d'événements (1, 2), caractérisé en ce qu'il comprend:

– une base de temps (3) dont la sortie est reliée à l'entrée d'un premier compteur (5.1) d'un premier ensemble de compteurs (5.1 à 5.X) diviseurs par 10, et montés en cascade, chacun de ces compteurs présentant, d'une part, une borne de débordement reliée à la borne d'entrée de comptage du compteur suivant et, d'autre part, une borne de remise à zéro,

– associé à chacun desdits compteurs dudit premier ensemble de compteurs (5.1 à 5.X), un circuit de détection (20, 21) détectant le signal de débordement de son compteur associé, ledit circuit de détection ayant une borne de remise à zéro,

– un premier circuit de retard (6) reliant ladite source d'événements (1, 2) à la borne de remise à zéro dudit circuit de détection (20, 21),

– un ensemble de portes de validation (10.1 à 10.X), respectivement affectées aux compteurs (5.1 à 5.X) dudit premier ensemble, chaque porte de validation ayant une entrée reliée à la sortie du circuit de détection correspondant et une autre entrée reliée à la source d'événements (1, 2),

– un second ensemble de compteurs (11$^1$.1 à 11$^1$.X, 11$^2$.1 à 11$^2$.(X–1), ...11$^X$.1) diviseurs par 10, dont les entrées de comptage sont reliées aux sorties dudit ensemble de portes de validation (10.1 à 10.X), chacun des compteurs dudit second ensemble de compteurs ayant une borne de remise à zéro et une sortie connectée à une mémoire (23) respective,

– un séquenceur (4) dont l'entrée est reliée à la base de temps (3) et dont la sortie est connectée à des moyens (8) de transfert du contenu de chaque compteur dudit second ensemble de compteurs dans sa mémoire (23) respective,

– des moyens de lecture (12.1 à 12.X) du contenu de chaque mémoire, les sorties desdits moyens de lecture étant dirigées vers un appareil d'enregistrement (13) assurant l'affichage dudit contenu,

– un second circuit de retard (9) dont l'entrée est reliée à la sortie dudit séquenceur (4) et dont la sortie est reliée aux bornes de remise à zéro des compteurs dudit premier ensemble de compteurs et aux bornes de remise à zéro des compteurs dudit second ensemble de compteurs.

2) Dispositif suivant la revendication 1, caractérisé en ce que lesdits moyens de lecture comprennent un ensemble de multiplexeurs (12.1 à 12.X) dont les entrées sont sélectivement reliées aux sorties desdites mémoires des compteurs par dix (11$^1$.1 à 11$^X$.1) sous la commande d'un circuit de commande d'enregistrement (15) comportant un circuit de séquencement dont la sortie est reliée en parallèle aux entrées de commande des multiplexeurs (12.1 à 12.X) et dont l'entrée est reliée à la sortie du second circuit à retard (9).

## Claims

1) A device for obtaining a time-distance histogram of successive events that are delivered from a source of events (1, 2), characterized in that it is comprised of:

– a time base (3) whose output is connected to a first counter (5.1) within a first set of cascaded decade counters (5.1–5.X), each of which having, on the one hand, an overflow terminal connected to the counting input of the next counter and, on the other hand, a reset terminal,

– a detection circuit (20, 21) associated to each counter belonging to said first set of counters (5.1–5.X) for detecting the overflow signal delivered from the associated counter, said detection circuit having a reset input,

– a first delay circuit (6) connected from said source of events (1, 2) to the reset input of said detection circuit (20, 21),

– a set of validation gates (10.1–10.X) respectively associated to counters (5.1–5.X) belonging to said first set of counters, each validation gate having one input connected from the output of the corresponding detection circuit and another input connected from the source of events (1, 2),

– a second set of decade counters (11$^1$.1–11$^1$.X, 11$^2$.1–11$^2$.(X–1), ...11$^X$.1) whose counting inputs are connected from the outputs of said set of validation gates (10.1–10.X), each counter belonging to said second set of counters having a reset input and an output connected to an associated memory (23),

– a timer (4) whose input is connected from the time base (3) and output is connected to transfer means (8) transferring the contents of each counter belonging to said second set of counters into said associated memory (23),

– read-out means (12.1–12.X) for reading the contents of each memory out, outputs of said read-out memory being transferred to a recording instrument (13) displaying said contents,

– a second delay circuit (9) whose input is connected from the output of said timer (4),and output is connected to reset terminals of counters belonging to said first set of counters and to reset terminals of counters belonging to said second set of counters.

2) A device according to claim 1, characterized in that said read-out means include a set of multiplexers (12.1–12.X) whose inputs are selectively connected to outputs of said memories of decade counters (11$^1$.1–11$^X$.1) under control of a recording control circuit (15) having a timing circuit whose output is parallel connected to control inputs of multiplexers (12.1–12.X) and input is connected from output of said second delay circuit.

## Patentansprüche

1. Schaltung zur Erlangung eines Zeit-Abstand-Histogramms aufeinanderfolgender Ereignisse von einer Quelle von Ereignissen (1, 2), dadurch gekennzeichnet, dass diese besteht aus:

– einer Zeitbasis (3), deren Ausgang mit dem Eingang eines ersten Zählers (5.1) einer ersten Gruppe von in Kaskade angeordneten Dekadenzählern (5.1–5.X) verbunden ist, von denen jeder einerseits eine Überlaufklemme, die mit dem Zähleingang des nächsten Zählers verbunden ist, und anderseits eine Rückstellklemme aufweist,

– einer Detektorschaltung (20, 21), die jedem zu der genannten ersten Gruppe von Zählern gehörenden Zähler zugeordnet ist und das von ihrem zugeordneten Zähler gelieferte Überlaufsignal feststellt, wobei die genannte Detektorschaltung eine Rückstellklemme besitzt,

– einer ersten Verzögerungsschaltung (6), die die genannte Quelle der Ereignisse (1, 2) mit dem Rückstelleingang der genannten Detektorschaltung (20, 21) verbindet,

– einer Gruppe von Validationstoren (10.1–10.X), die jeweils zu der ersten Gruppe von Zählern gehörenden Zählern (5.1–5.X) zugeordnet sind, wobei jedes Validationstor einen Eingang, der mit dem Ausgang der entsprechenden Detektorschaltung verbunden ist, und einen anderen Eingang, der mit der Quelle der Ereignisse verbunden ist, besitzt,

– einer zweiten Gruppe von Dekadenzählern (11¹.1–11¹.X, 11².1–11².X–1, ...11$^X$.1), deren Zähleingänge mit den Ausgängen der genannten Gruppe von Validationstoren (10.1–10.X) verbunden sind, wobei jeder Zähler der zweiten Zählergruppe eine Rückstellklemme und einen mit einem zugeordneten Speicher (23) verbundenen Ausgang besitzt,

– einem Sequenzer (4), dessen Eingang mit der Zeitbasis (3) verbunden ist, und dessen Ausgang mit Übertragungsmitteln (8) verbunden ist, die den Inhalt jedes zu der zweiten Gruppe von Zählern gehörenden Zählers in den zugeordneten Speicher (23) übertragen,

– Lesemitteln (12.1–12.X) zum Lesen des Inhalts jedes Speichers, wobei die Ausgänge der genannten Lesemittel einem Aufzeichnungsgerät (13) zugeleitet werden, das eine Anzeige des genannten Inhalts gewährleistet,

– einer zweiten Verzögerungsschaltung (9), deren Eingang mit dem Ausgang des genannten Sequenzers (4) verbunden ist, und deren Ausgang mit Rückstellklemmen von Zählern, die zur ersten Gruppe von Zählern gehören und mit Rückstellklemmen von Zählern, die zur zweiten Gruppe von Zählern gehören, verbunden ist.

2. Schaltung nach Anspruch 1, dadurch gekennzeichnet, dass die genannten Lesemittel eine Gruppe von Multiplexern (12.1–12.X) enthalten, deren Eingänge selektiv mit Ausgängen der genannten Speicher von Dekadenzählern (11¹.1–11$^X$.1) unter Steuerung durch eine Aufzeichnungs-Steuerschaltung (15) verbunden sind, die eine Folgeschaltung enthält, deren Ausgang parallel mit Steuereingängen und Multiplexern (12.1–12.X) verbunden ist, und deren Eingang mit dem Ausgang der zweiten Verzögerungsschaltung (9) verbunden ist.

# FIG.1

0 046 110

FIG.2

0 046 110

# FIG.3

FIG.4

FIG.5

FIG.6

13

# FIG.7

0 046 110